# EUROPEAN PATENT SPECIFICATION

(11) **EP 0 391 959 B1**
(45) Date of publication and mention of the grant of the patent: **27.01.1993**
(21) Application number: 89900765.2
(22) Date of filing: 12.12.1988
(51) Int. Cl.: A61J 3/10, A61K 9/44

(54) **TRI-SCORED DRUG TABLET**
DREIFACH GEKERBTE ARZNEIMITTELTABLETTE
COMPRIME PHARMACEUTIQUE A TROIS ENCOCHES

(30) Priority: 17.12.1987 US 134006
(43) Date of publication of application: 17.10.1990
(73) Proprietor: THE UPJOHN COMPANY, Kalamazoo, Michigan 49001 (US)
(72) Inventor: NI, Phillip, F., Mattawan, MI 49071 (US); ODAR, Larry, F., Galesburg, MI 49053 (US)
(74) Representative: Perry, Robert Edward
(86) International application number: US8804367
(87) International publication number: WO8905624

(56) References cited:
- DE-U- 8 632 046
- GB-A- 993 291
- GB-A- 2 057 878
- US-A- 2 052 376

## Description

### FIELD OF THE INVENTION

This invention relates to a tri-scored drug tablet and, more particularly, to a tri-scored drug tablet having a length greater than its width and structure for facilitating a breaking of the tablet into four equal parts.

### BACKGROUND OF THE INVENTION

Scored drug tablets have been known for many years and are provided to patients to enable them to break the tablet into two or more parts to enable fractional dosages of the medicine to be taken by the patient (see British-Patent No. 993 291). Heretofore, problems have been encountered by the patient in facilitating a proper breakage of the tablet into its component parts due to the strength of the binder agents utilized to bind the active pharmacological agents contained within the tablet. Arthritic patients may be unable to break the tablet into its component parts due to the aforesaid strength characteristic. In some instances, a sharp edged tool, such as a knife, is needed in order to effect an even breakage of the tablet into its component parts. Further, and more important, is the requirement that the fractured tablet components contain the corresponding fraction, within the required ±15% limits, of active pharmacological agents in each of the divided sections.

DE-U-8 632 046 discloses a tri-scored drug tablet, the elongated body of which has a length greater than is width, said body has top and bottom surfaces, said top surface being flat,and aligned breaking grooves are formed in said top and bottom surface, each said breaking groove extending laterally across the width of said tablet.

It is an object of the present invention to provide a tri-scored drug tablet capable of being easily broken into four parts.

It is a further object of this invention to provide a tri-scored drug tablet, as aforesaid, capable of being broken into its component parts by simply holding the tablet between the thumb and forefinger and applying the requisite compressive breaking force.

It is a further object of this invention to provide a tri-scored drug tablet, as aforesaid, which has a length greater than its width and appropriate structure at its opposite longitudinal ends and longitudinally central part as well as structure defining a zone of weakness at the longitudinally central part and in the central region of each half section to facilitate a breakage of the tablet into separate but equal components.

It is a further object of this invention to provide a tri-scored drug tablet, as aforesaid, having the requisite strength characteristics to prevent premature breakage of the tablet either during manufacture, insertion into a container or during transit of the container to the end user.

It is a further object of this invention to provide a tri-scored drug tablet, as aforesaid, which is easy to manufacture and is of sufficient but yet minimum size to facilitate easy swallowing of the tablet by the end user patient.

### SUMMARY OF THE INVENTION

The objects and purposes of the broadest aspect of the invention, including those set forth above, are met by providing a tri-scored drug tablet having an elongated tablet body with a length greater than its width. The tablet body also has a bottom surface with a pair of concavities being provided therein. Aligned breaking grooves are formed in the top and bottom surfaces, The breaking grooves in the bottom surface are provided in both of the concavities as well as at a longitudinally central part and extend laterally across the width of the tablet at an apex of each cavity and at the longitudinally central part. Thus, upon a user grasping both ends of the tablet between a forefinger and a thumb on each hand, a force is applied to effect a fracture of the tablet along the breaking groove at the longitudinally central part to divide the tablet into two.half sections, the user thereafter placing the two half sections in a stacked arrangement so that the two concavities face one another and the thickest dimensioned portions at the longitudinal ends of the half sections are in engagement, the user thereafter applying a compressive force to the midpoint of the stacked half sections to effect a fracture of each of the halves along the breaking grooves at the apexes of the concavities to divide each of the half sections into two quarter sections.

### BRIEF DESCRIPTION OF THE DRAWING

The subject matter of the invention will be described in more detail hereinafter in connection with the exemplary embodiments illustrated in the drawing, in which:
Figure 1 is a left top perspective view of a tri-scored tablet embodying the invention;
Figure 2 is a left bottom perspective view of the tablet;
Figure 3 is a top view thereof;
Figure 4 is a bottom view thereof;
Figure 5 is a side view thereof;
Figure 6 is an end view thereof;
Figure 7 is a sectional view taken along the line VII-VII of Figure 5;
Figure 8 is a sectional view taken along the line VIII-VIII of Figure 5;
Figure 9 is a side view similar to Figure 5 but with the tablet separated into two half sections;
Figure 10 is a side view of a half section resting on a support surface;
Figure 11 is a side view of two half sections, one stacked on the other;
Figure 12 is a side view of the stacked half sections following the application of force causing a simultaneous breakage of each half section into two quarter sections;
Figure 13 illustrates a half section having an arcuate end, so that the opposing ends of the half sections will engage along a line;
Figure 14 illustrates a half section having a flat end surface, so that the opposing ends of the half section will engage along or at opposing flat surfaces; and
Figure 15 illustrates a half section having a pair of laterally spaced pads with flat surfaces, so that the opposing ends of the half sections will engage at the opposing pads.

### DETAILED DESCRIPTION

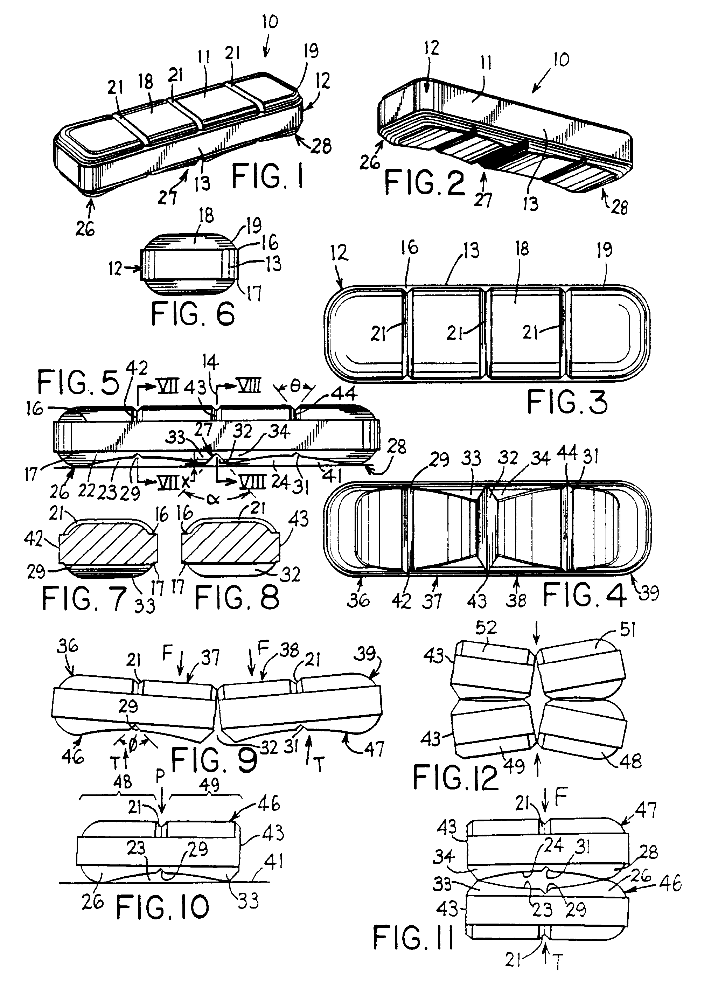

A tri-scored drug tablet 10 is illustrated in a perspective view in Figures 1 and 2. Figure 1 illustrates the drug tablet from the top and left end thereof. Figure 2 illustrates the drug tablet from the bottom and left end thereof. The tablet 10 has an elongated tablet body 11 having a length greater than its width. The tablet body includes an elongated central body part 12 of a finite and uniform thickness and having an outwardly facing, smooth and uninterrupted perimetrical surface 13 extending parallel to a theoretical line 14 (Figure 5) extending through a geometric center of the tablet and parallel to the section line VIII-VIII in Figure 5. Further characteristics of the theoretical line will be explained in more detail below. The central body part 12 is generally of an oblong, almost rectangular sharp with rounded corners as shown in Figures 3 and 4. Further, the central body part 12 has a flat top surface 16 and a flat bottom surface 17.

The top surface 16 of the central body part 12 has a layer 18 of material of uniform thickness provided thereon along the length thereof. The upper peripheral edges of the layer 18 are rounded as at 19. Three laterally extending scores or breaking grooves 21 are provided in the layer 18 at equidistantly spaced intervals along the length of the tablet to divide the layer 18 into four equal sections. The upper surface of the layer 18 is flat and parallel to the surface 16. The scores or breaking grooves 21 have a depth that is approximately one fourth to one half the thickness of the layer 18, preferably one third the thickness. Further, the breaking grooves have an arcuate contour conforming to the rounded peripheral edge at both ends of the breaking grooves. The included angle ϑ between the sidewalls of the breaking grooves 21 is in the range of 70° to 110°, preferably 90°.

The bottom surface of the central body part 12 has a layer 22 of material provided thereon along the length thereof. Into the bottom facing surface of the layer 22 there is provided a pair of arcuately contoured concavities 13 and 24. The provision of the arcuately contoured concavities 13 and 24 in the bottom facing surface of the layer 22 leaves three raised surface portions 26, 27 and 28 at the opposite longitudinal ends of each concavity. The downwardly facing surface of each arcuately contoured concavity 23 and 24 is smooth between the raised surfaces 26, 27, and 27, 28 except for the provision of a breaking groove 29 in the concavity 23 and a breaking groove 31 in the concavity 24. Each breaking groove 29 and 31 extends laterally of the tablet in a direction perpendicular to the longitudinal axis of the drug tablet 10. Further, the breaking grooves 29 and 31 are both located at the apex of the arcuately contoured concavities 23 and 24 and the depth of each groove extends to but not beyond the bottom surface 17 of the central body part 12 as illustrated in Figure 5.

The raised surface portion 27 in the central region of the bottom facing surface also has a breaking groove 32 therein that extends laterally of the tablet parallel to the breaking grooves 29 and 31. The breaking groove 32 divides the raised surface portion 27 into two raised parts 33 and 34 of equal proportion. The breaking groove 32 has a depth such that the apex of the V-like bottom is spaced approximately 0,0127 cm (.005 inches) from the bottom surface 17 of the central body part 12 as illustrated in Figure 5. The included angle φ (Figure 9) between the sidewalls of the breaking grooves 29 and 31 is in the range of 70° to 110°, preferably 90°. The included angle α (Figure 5) between the sidewalls of the breaking groove 32 is in the range of 60° to 100°, preferably 80°.

Each of the three breaking grooves 29, 31 and 32 are aligned with a respective one of the three breaking grooves 21 so that vertical cutting planes perpendicular to the longitudinal axis of the tablet and extending through each aligned pair of breaking grooves 21, 29; 21, 32; and 21, 31 will divide the tablet into four equal sections 36, 37, 38 and 39.

When the tablet 10 is placed so that the raised surface portions 26 and 28 rest on a flat surface 41 (Figure 5), a gap or spacing X exists between the raised parts 33 and 34 and the flat surface 41. This space or gap is insufficient, however, for facilitating a fracturing of the tablet along the aligned breaking grooves 21, 32 when a force is applied to the upper surface of the layer 18 at the central breaking groove 21.

The spacing between the bottom of each of the breaking grooves 21 and the bottom of the aligned one of the breaking grooves 29, 31 and 32 define a zone of weakness generally referred to by the reference numerals 42, 43 and 44. A cross section of the zones of weakness 42 and 43 are shown in Figures 7 and 8. Thus, when the tablet is grasped by a user between the thumb and forefinger of each hand, an appropriate force can be applied to effect a fracturing of the tablet along the zone of weakness 43, namely, along a plane defined by the aligned breaking grooves 21 and 32. This is schematically represented in Figure 9. Following a separating of the tablet 10 into two half sections 46 and 47, one of the half sections, such as the half section 46 in Figure 10, can be positioned so that the raised surfaces 26 and 33 rest on a supporting surface 41. In other words, the concavity 23 faces the supporting surface 41. A pencil or finger of the user can then be used to apply a force in direction of the arrow P to the aligned breaking grooves 21 and 29 to effect a fracturing of the half section into two quarter sections 48 and 49.

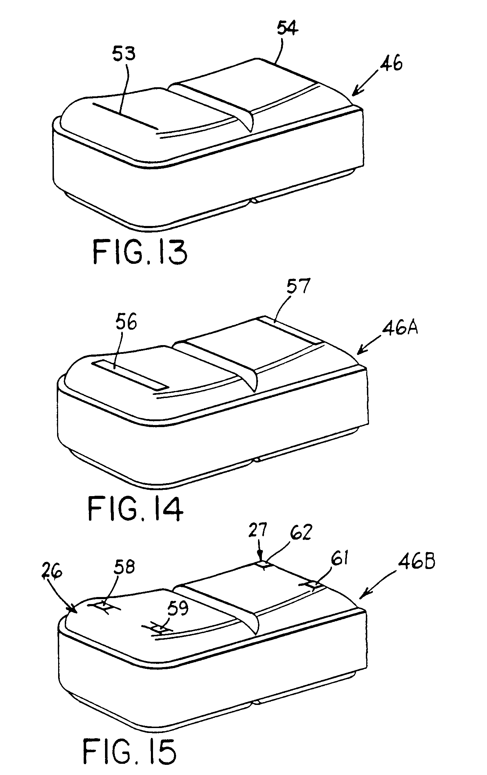

In the alternative, the half sections 46 and 47 can be simultaneously fractured into quarter sections utilizing a different methodology. For example, and referring to Figure 11, the half sections 46 and 47 are positioned so that the raised surfaces 26 and 33 engage respectively the raised surfaces 28 and 34. In other words, the concavities 23 and 24 face one another. In addition, the aligned set of breaking grooves 21 and 29 on the half section 46 are parallel to and congruent with the aligned set of breaking grooves 21 and 31 on the half section 46. A placement of this stacked arrangement between the thumb and forefinger of a user's hand will, upon the application of a compressive or pinching force in the direction of the arrows F and T will effect a simultaneous fracturing of the half sections 46 and 47 into four quarter sections 48, 49, 51 and 52.

During each of the aforementioned fractures, a fracturing of the tablet 10 into half sections 46 and 47 results in the tablet having the requisite active pharmacological agents in each half and within the requisite limits of ±15%. Similarly, a fracturing of the half sections 46 and 47 into quarter sections also results in each quarter section having the requisite active pharmacological agent within the prescribed ±15% limit.

Figures 13 to 15 illustrate a half section, such as the half section 46. In order to differentiate between each of the half sections illustrated in these figures, the reference numeral 46 has been utilized to identify the half section in Figure 13 whereas the suffix "A" and "B" have been added to the reference numeral 46 in Figures 14 and 15. In the embodiment illustrated in Figure 13, a pair of lines 53 and 54 are illustrated and represent the lines whereat engagement of the half section 46 occurs with either a flat surface 41 or the other half section 47 when in a stacked arrangement, such as is illustrated in Figure 11. Both half sections would, therefore, engage at lines congruent with the lines 53 and 54 illustrated in Figure 14.

In the embodiment of Figure 14, the half section 46A has a pair of flat surface areas 56 and 57 thereon. Thus, the half section 46A can be oriented so that the flat surfaces 56 and 57 rest on the flat surface 41 or corresponding flat surfaces of the other half section when the half sections are positioned in a stacked arrangement, such as illustrated in Figure 11.

In the embodiment illustrated in Figure 15, four surface pads 58, 59, 61 and 62 are provided at the raised surface portions 26 and 27. The surface pads 58 are flat surfaces that are raised above the upper most height of the raised surface portions 26 and 27. Thus, the surface pads 58, 59, 61 and 62 will rest on a flat surface when the half section is placed in the position illustrated in Figure 10. Similarly, the aforementioned surface pads will rest on corresponding surface pads of the other half section when the half sections are stacked in the arrangement such as illustrated in Figure 11.

Although particular preferred embodiments of the invention have been disclosed in detail for illustrative purposes, it will be recognized that variations or .modifications of the disclosed tablet lie within the scope of the present invention.

## Claims

1. An elongate tri-scored drug tablet (10) having a flat top surface, a pair of concavities (23,24) in its bottom surface, and aligned breaking grooves (21; 29,31,32) formed in the top and bottom surfaces, such that the tablet can be broken into half-sections that themselves can be broken into quarter-sections,
wherein the concavities are equal in size and have parallel major and minor axes, and each concavity has a smooth and uninterrupted arcuate surface extending between a longitudinally central part (27) and a respective end of the tablet, such that the tablet is thicker at its ends (26,28) and at the longitudinally central part than at the apex of each concavity; and
wherein the breaking grooves in the bottom surface of the tablet extend laterally across the width of the tablet, and are at each said apex and at the longitudinally central part.

2. A tablet according to claim 1, wherein the body includes, across its thickness, a uniformly-thick central body part (12) having top and bottom surfaces (16,17) that extend around the periphery of the tablet, and a smooth and uninterrupted circumferential surface (13) perpendicular to the top surface of the tablet;
wherein the depth of each concavity does not extend as far as the bottom peripheral surface;
wherein the depth of each breaking groove (29,31,32) in the bottom surface of the tablet extends to the bottom peripheral surface; and
wherein the depth of each breaking groove (21) in the top surface of the tablet extends one-fourth to one-half the distance between that top surface and the top peripheral surface.

3. A tablet according to claim 2, wherein the depth of each breaking groove in the top surface of the tablet extends one-third of the distance between that top surface and the top peripheral surface.

## Patentansprüche

1. Langgestreckte, dreifach gekerbte Arzneimitteltablette (10) mit einer flachen Oberseite, zwei Konkavitäten (23, 24) in ihrer Unterseite sowie aufeinander ausgerichteten, in ihren Ober- und Unterseiten geformten Bruchrillen (21; 29, 31, 32), so daß die Tablette in Hälften brechbar ist, die ihrerseits in Viertel(teile) brechbar sind,
wobei die Konkavitäten gleiche Größen besitzen und parallele Haupt- und Nebenachsen aufweisen und jede Konkavität eine glatte und ununterbrochene, zwischen einem in Längsrichtung zentralen Teil (27) und einem jeweiligen Ende der Tablette verlaufende gekrümmte oder Bogen-Fläche aufweist, so daß die Tablette an ihren Enden (26, 28) und an dem in Längsrichtung zentralen Teil dicker ist als am Scheitelpunkt jeder Konkavität, und
wobei die Bruchrillen in der Unterseite der Tablette quer über die Breite der Tablette verlaufen und an jedem Scheitelpunkt sowie an dem in Längsrichtung zentralen Teil liegen.

2. Tablette nach Anspruch 1, wobei der Körper über seine Dicke hinweg einen gleichmäßig dicken zentralen Körperteil (12) mit Ober- und Unterseiten (16, 17), die um den Umfang der Tablette herum verlaufen, und eine senkrecht zur Oberseite der Tablette liegende glatte und ununterbrochene Umfangsfläche (13) aufweist,
wobei die Tiefe jeder Konkavität nicht weiter als bis zur unteren Umfangsfläche reicht,
wobei die Tiefe jeder Bruchrille (29, 31, 32) in der Unterseite der Tablette bis zur unteren Umfangsfläche reicht und
wobei die Tiefe jeder Bruchrille (21) in der Oberseite der Tablette von einem Viertel bis zur Hälfte der Strecke zwischen dieser Oberseite und der oberen Umfangsfläche reicht.

3. Tablette nach Anspruch 2, wobei die Tiefe jeder Bruchrille in der Oberseite der Tablette einem Drittel der Strecke zwischen dieser Oberseite und der oberen Umfangsfläche entspricht.

## Revendications

1. Comprimé pharmaceutique allongé (10) à trois encoches ayant une surface supérieure plate, deux concavités (23, 24) dans sa surface inférieure et des gorges alignées (21; 29, 31, 32) de rupture formées dans les surfaces supérieure et inférieure, de manière que le comprimé puisse être rompu en moitiés qui peuvent elles-mêmes être rompues en quarts,
dans lequel les concavités sont de dimensions égales et ont des grand et petit axes parallèles, et chaque concavité présente une surface incurvée lisse et ininterrompue s'étendant entre une partie longitudinalement centrale (27) et une extrémité respective du comprimé, de manière que le comprimé soit plus épais à ses extrémités (26, 28) et à la partie longitudinalement centrale qu'au sommet de chaque concavité ; et
dans lequel les gorges de rupture dans la surface inférieure du comprimé s'étendent latéralement sur la largeur du comprimé et sont à chacun desdits sommets et à la partie longitudinalement centrale.

2. Comprimé selon la revendication 1, dans lequel le corps comprend, à travers son épaisseur, une partie centrale (12) de corps d'épaisseur uniforme ayant des surfaces supérieure et inférieure (16, 17) qui s'étendent le long de la périphérie du comprimé, et une surface circonférentielle lisse et ininterrompue (13) perpendiculaire à la surface supérieure du comprimé ;
dans lequel la profondeur de chaque concavité ne s'étend pas aussi loin que la surface périphérique inférieure ;
dans lequel la profondeur de chaque gorge de rupture (29, 31, 32) dans la surface inférieure du comprimé s'étend jusqu'à la surface périphérique inférieure ; et
dans lequel la profondeur de chaque gorge de rupture (21) dans la surface supérieure du comprimé s'étend sur une distance comprise entre un quart et la moitié de la distance entre cette surface supérieure et la surface périphérique supérieure.

3. Comprimé selon la revendication 2, dans lequel la profondeur de chaque gorge de rupture dans la surface supérieure du comprimé s'étend sur un tiers de la distance entre cette surface supérieure et la surface périphérique supérieure.
